# FASCICULE DE BREVET EUROPEEN

(11) **EP 2 300 424 B1**
(45) Date de publication et mention de la délivrance du brevet: **18.01.2012**
(21) Numéro de dépôt: 09784501.0
(22) Date de dépôt: 09.07.2009
(51) Int. Cl.: C07D 209/12, A61P 25/16, A61P 25/28, C07D 209/18, A61K 31/405

(54) **UTILISATION DE DERIVES D'INDOLE COMME ACTIVATEURS DE NURR-1, POUR LE TRAITEMENT DE LA MALADIE DE PARKINSON**
VERWENDUNG VON INDOLDERIVATEN ALS NURR-1-AKTIVATOREN ZUR BEHANDLUNG VON MORBUS PARKINSON
USE OF INDOLE DERIVATIVES AS NURR-1 ACTIVATORS FOR TREATING PARKINSON S DISEASE

(30) Priorité: 10.07.2008 FR 0854712
(43) Date de publication de la demande: 30.03.2011
(73) Titulaire: Laboratoires Fournier S.A., 21000 Dijon (FR)
(72) Inventeur: BOUBIA, Benaïssa, F-21850 Saint Apollinaire (FR); VAN VLIET, Bernard, Johannes, NL-1216 SZ Hilversum (NL); DEN HARTOG, Jacobus, Antonius, Joseph, NL-1382 TA Weesp (NL); McCREARY, Andrew, NL-1312 Et Almere (NL); TALLANDIER, Mireille, F-21490 Bretigny (FR); VAN DONGEN, Maria, Johanna, Petronella, NL-1213 BW Hilversum (NL); POUPARDIN-OLIVIER, Olivia, F-21490 Varois Et Chaignot (FR)
(74) Mandataire: Hubert, Philippe
(86) Numéro de dépôt international: PCT/FR2009/051372
(87) Numéro de publication internationale: WO 2010/004221

(56) Documents cités:
- WO-A-03/015780
- WO-A-2005/056522
- WO-A-2008/034974
- FR-A- 2 890 071
- DUBOIS CELINE ET AL: "Identification of a potent agonist of the orphan nuclear receptor Nurr1." CHEMMEDCHEM SEP 2006, vol. 1, no. 9, septembre 2006 (2006-09), pages 955-958, XP007907069 ISSN: 1860-7179

## Description

La présente invention concerne une nouvelle utilisation en thérapeutique de certains dérivés d'indole dans le traitement et/ou la prévention de maladies impliquant les récepteurs nucléaires NURR-1. Plus spécifiquement, cette invention concerne l'utilisation de ces composés pour la fabrication d'un médicament pour le traitement et/ou la prévention de la maladie de Parkinson.

### Art antérieur

Les maladies neurodégénératives sont définies comme des maladies caractérisées par un dysfonctionnement progressif du système nerveux. Elles sont souvent associées à une atrophie des structures du système nerveux central ou périphérique touché. Elles incluent, entre autres, des maladies telles que la maladie d'Alzheimer, la maladie de Creutzfeldt-Jakob, la maladie de Huntington, la maladie de Parkinson, les maladies lysosomales, la paralysie supranucléaire progressive, la sclérose en plaques et la sclérose latérale amyotrophique. Parmi les maladies neurodégénératives, la maladie de Parkinson est une affection qui touche environ quatre millions de personnes dans le monde. Bien qu'elle affecte des individus de tout âge, elle est plus commune chez les personnes âgées (avec 2 % de la population des personnes de plus de 65 ans touchées par cette maladie). Elle est caractérisée par une dégénérescence des neurones dopaminergiques de la substantia nigra.

La dopamine est un neurotransmetteur qui exerce un rôle central dans le contrôle des mouvements volontaires, les fonctions cognitives et le développement de comportements associés aux émotions.

La stratégie thérapeutique actuelle pour le traitement de la maladie de Parkinson repose sur l'atténuation des symptômes en suppléant la déficience en dopamine par l'administration d'un précurseur métabolique tel que la L-DOPA.

Or, aujourd'hui, l'augmentation de la fréquence de cette pathologie a rendu nécessaire le développement de nouveaux agents thérapeutiques, exerçant un rôle bénéfique dans la survie et la différenciation neuronale.

Ce développement a conduit à identifier des composés capables d'activer les récepteurs nucléaires impliqués dans la pathogénèse de la maladie de Parkinson.

Fortement exprimé dans le cerveau, le facteur de transcription NURR-1, membre de la superfamille des récepteurs nucléaires orphelins, a été identifié comme ayant un rôle essentiel dans le développement et le maintien des neurones dopaminergiques du mésencéphale (Zetterstrom, Solomin et al. 1997, Science. 1997 Apr 11;276(5310):248-50).

Le récepteur nucléaire NURR-1 intervient dans le maintien du phénotype dopaminergique via la régulation des gènes spécifiques des neurones dopaminergiques (DA). Il favorise aussi la survie des neurones DA en les protégeant des agressions toxiques. Le récepteur nucléaire NURR-1 sert donc de facteur de transcription spécifique des neurones dopaminergiques pour lequel les activités pourraient être régulées en modulant la neurotransmission dopaminergique dans la maladie de Parkinson.

Ce récepteur se lie à l'ADN sous forme de monomères, d'homodimères ou d'hétérodimères avec RXR (Retinoid X Receptor) un récepteur nucléaire qui est l'hétéropartenaire de nombreux autres membres de la famille des récepteurs nucléaires. RXR intervient dans de nombreux processus physiologiques comme le métabolisme des lipides et du glucose, le développement et la différenciation. NURR-1 interagit ainsi avec les isoformes α et γ de RXR. RXRα est exprimé de façon ubiquitaire alors que l'expression de RXRγ se concentre principalement dans le cerveau et notamment dans le striatum, l'hypothalamus et l'hypophyse.

Les complexes formés NURR-1/RXRα et NURR-1/RXRγ sont capables de réguler la transcription en réponse à un ligand de RXR. RXR module donc positivement le potentiel d'activation de la transcription de NURR-1.

L'identification de composés capables d'induire l'activité des complexes NURR-1/RXRα et NURR-1/RXRγ devrait en conséquence permettre de disposer de nouvelles voies pour traiter la maladie de Parkinson.

On connaît par le document WO2003/015780 des composés hétérocycliques actifs pour le traitement de la maladie de Parkinson.

Par ailleurs, les documents WO2004/072050, FR 2 903 105, FR 2 903 106 et FR 2 903 107 décrivent des composés activateurs du récepteur NURR-1, tandis que l'utilisation de composés hétérocycliques modulateurs de l'activité des récepteurs de la famille des NGFI-B (dont NURR-1 est un membre) est décrite dans le document WO2005/047268.

Enfin, on connaît par le document WO2005/056522 des dérivés de l'indole qui sont des activateurs des récepteurs nucléaires PPAR et trouvent application en tant que principes actifs de médicaments pour le traitement de certaines maladies du système cardiovasculaire.

Dans ce contexte, il a été découvert, et ceci constitue le fondement de la présente invention, que certains composés dérivés de l'indole couverts par la formule générale mentionnée dans le document WO2005/056522 sont des agonistes sélectifs NURR-1/RXRα et NURR-1/RXRγ, capables d'inhiber la dégénérescence des neurones observée dans la maladie de Parkinson.

Ainsi, il a été montré que, de façon surprenante, les composés de l'invention présentent, en plus de leur pouvoir activateur PPAR, un très fort potentiel d'activation des hétérodimères NURR-1/RXRα et NURR-1/RXRγ. Ces composés, en raison de leurs propriétés uniques, sont de ce fait particulièrement intéressants pour leur utilisation dans le traitement ou la prévention des maladies dans lesquelles le récepteur NURR-1 est impliqué, notamment des maladies neurodégénératives et en particulier de la maladie de Parkinson.

Ainsi, selon un premier aspect, la présente invention concerne, en tant que produits nouveaux, les composés dérivés de l'indole choisis parmi
i) les composés de formule : 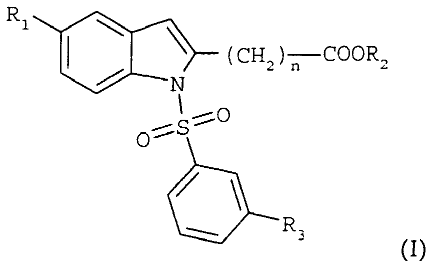 dans laquelle :
   R₁ représente un halogène ou un groupe trifluorométhyle,
   R₂ représente un atome d'hydrogène ou un groupe alkyle en C₁-C₄,
   R₃ représente un groupe isopropyle (1-méthyléthyl) ou un groupe *tertio*-butyle (1,1-diméthyléthyl),
   n = 3 ou 4
ii) les sels pharmaceutiquement acceptables desdits composés de formule (I).

Il a été constaté, et ceci constitue l'originalité des composés de l'invention, que la présence simultanée :
- d'un substituant isopropyle ou d'un substituant *tertio*-butyle en position méta sur le groupement benzènesulfonyle ; et
- d'un halogène ou d'un groupe trifluorométhyle en position 5 de l'indole confère aux composés de l'invention une activité remarquable et tout à fait inattendue vis-à-vis des récepteurs NURR-1.

Les composés de l'invention présentent donc une structure chimique qui, bien que généralement couverte par la formule générale décrite dans le document WO 2005/056522, résulte d'une sélection que n'aurait pu opérer un homme du métier à la recherche de composés destinés au traitement de la maladie de Parkinson.

Selon un deuxième aspect, l'invention concerne les composés précités pour leur utilisation en tant que substances pharmacologiquement actives, ainsi que les compositions pharmaceutiques les contenant.

Selon un troisième aspect, l'invention concerne l'utilisation d'au moins un composé de formule (I) ou l'un de ses sels pharmaceutiquement acceptable en tant que principe actif pour la préparation d'un médicament destiné au traitement des maladies dans lesquelles le récepteur NURR-1 est impliqué, notamment des neuro-dégénérescences, comme en particulier la maladie de Parkinson.

### Description détaillée

Dans la présente description, on entend par groupe alkyle en C₁-C₄ une chaîne hydrocarbonée saturée, linéaire ou ramifiée, ayant de 1 à 4 atomes de carbone, et en particulier un groupe méthyle, éthyle, propyle, 1-méthyl-éthyle, butyle, 1-méthyl-propyle, 2-méthyl-propyle ou 1,1-diméthyl-éthyle.

Par halogène, on entend un atome de fluor ou de chlore.

Les composés de formule (I) dans laquelle R₂ représente un atome d'hydrogène sont des acides carboxyliques qui peuvent être utilisés sous la forme d'acides libres ou sous la forme de sels, lesdits sels étant obtenus par combinaison de l'acide avec une base minérale ou organique non toxique, de préférence pharmaceutiquement acceptable. Parmi les bases minérales, on peut utiliser par exemple les hydroxydes de sodium, de potassium, de magnésium ou de calcium. Parmi les bases organiques, on peut utiliser par exemple les amines, les aminoalcools, des acides aminés basiques tels que la lysine ou l'arginine ou encore des composés porteurs d'une fonction ammonium quaternaire tels que par exemple la bétaïne ou la choline.

Les composés selon l'invention peuvent être préparés selon un premier procédé consistant à :
a) faire réagir le composé de formule (II) 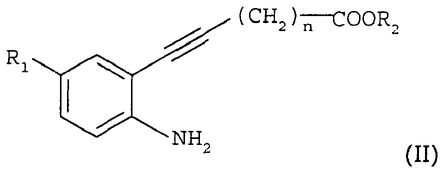 dans laquelle :
   R₁ représente un halogène ou un groupe trifluorométhyle,
   R₂ représente un atome d'hydrogène ou un groupe alkyle en C₁-C₄,
   n = 3 ou 4 ;
   avec un chlorure de benzènesulfonyle de formule (III) 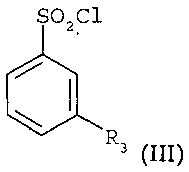
   dans laquelle :
   R₃ représente un groupe isopropyle ou *tertio-butyle,*
   en présence d'un solvant et d'une base, comme par exemple la pyridine, à température ambiante, pendant environ 15 heures, pour obtenir le composé de formule : 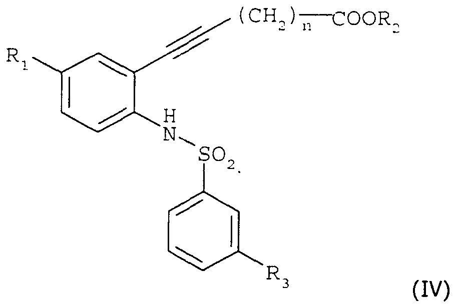 dans laquelle :
      R₁, R₂, R₃ et n conservent la même signification que dans les composés de départ ;
b) effectuer une cyclisation du composé de formule (IV), par exemple par action de l'acétate de cuivre II (voir par exemple J. Org. Chem., 2004, 69 (4), 1126-1136), dans un solvant tel que le 1,2-dichloroéthane à une température proche de la température de reflux du solvant, pendant environ 15 heures, pour obtenir le composé de formule 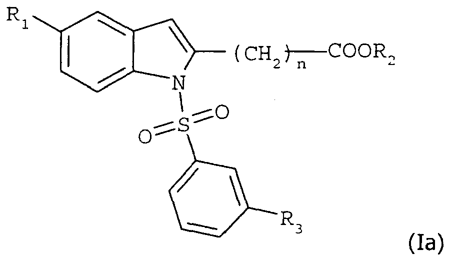 dans laquelle :
   R₁, R₂, R₃ et n conservent la même signification que dans le composé de départ ;
c) si nécessaire, hydrolyser la fonction ester du composé de formule (Ia), par exemple par action d'une base minérale telle que la lithine selon des modes opératoires bien connus de l'homme du métier, pour obtenir, après traitement acide, le composé de formule (I) sous sa forme d'acide libre :

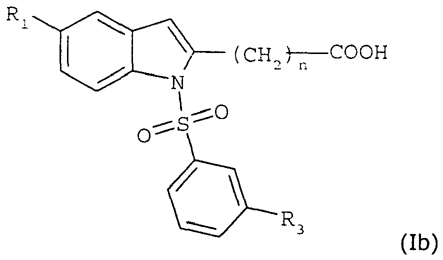

Selon une première variante, les composés de formule (I) peuvent être obtenus par un procédé consistant à :
a) cycliser le composé de formule 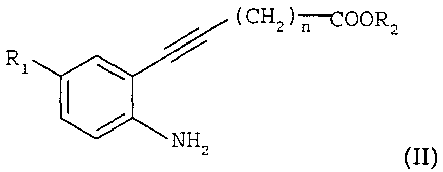 dans laquelle :
   R₁ représente un halogène ou un groupe trifluorométhyle,
   R₂ représente un atome d'hydrogène ou un groupe alkyle en C₁-C₄,
   n = 3 ou 4 ;
   dans des conditions analogues à celles décrites pour réaliser l'étape b) du procédé général précédent, pour obtenir le composé indolique de formule 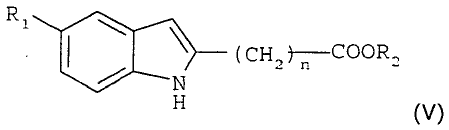
   dans laquelle :
   R₁, R₂ et n conservent la même signification que dans le composé de départ ;
b) faire réagir le composé de formule (V) avec un chlorure de benzènesulfonyle de formule (III) 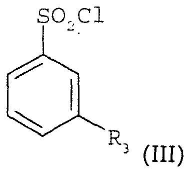 dans laquelle :
   R₃ représente un groupe isopropyle ou *tertio-*butyle*,*
   dans un solvant tel que par exemple le diméthylformamide (DMF), à température ambiante et pendant environ 3 heures après activation du composé indolique de formule (V) par l'hydrure de sodium, pour obtenir le composé de formule (Ia) 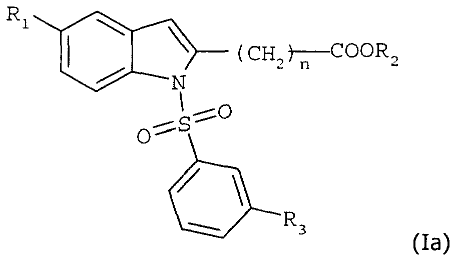
   dans laquelle :
   R₁, R₂, R₃ et n conservent la même signification que dans le composé de départ ;
c) hydrolyser, si nécessaire, la fonction ester du composé de formule (Ia), par exemple (dans le cas d'un ester *t*-butylique) par action d'un acide organique tel que l'acide trifluoroacétique, dans un solvant tel que le dichlorométhane, selon des modes opératoires bien connus de l'homme du métier, pour obtenir le composé de formule (I) sous sa forme d'acide libre :

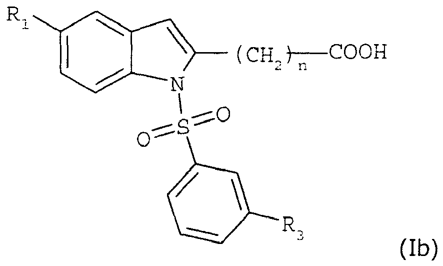

Selon une seconde variante, les composés de formule (I) peuvent être obtenus par un procédé consistant à :
a) faire réagir le composé de formule (VI) 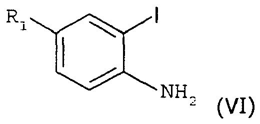 dans laquelle :
   R₁ représente un halogène ou un groupe trifluorométhyle ;
   avec un chlorure de benzènesulfonyle de formule (III) 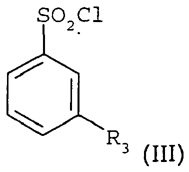
   dans laquelle :
   R₃ représente un groupe isopropyle ou *tertio-butyle,*
      dans un solvant tel que par exemple la pyridine, à température ambiante et pendant 4 heures, pour obtenir le composé de formule (VII) 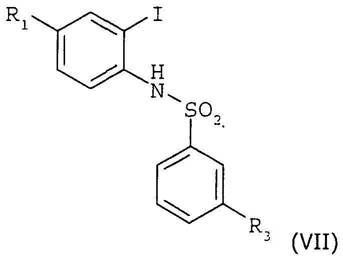
   dans laquelle :
   R₁ et R₃ conservent la même signification que dans les composés de départ ;
b) faire réagir le composé de formule (VII) avec un dérivé acétylénique de formule 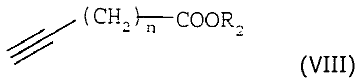 dans laquelle :
   R₂ représente un atome d'hydrogène ou un groupe alkyle en C₁-C₄,
   n = 3 ou 4 ;
   en présence d'iodure cuivreux, d'un catalyseur à base de palladium tel que par exemple le chlorure de bis(triphénylphosphine)palladium, et d'une base organique comme par exemple la triéthylamine, dans un solvant comme par exemple le diméthylformamide (DMF) à une température comprise entre la température ambiante et 80 °C pendant 12 heures, pour obtenir le composé de formule 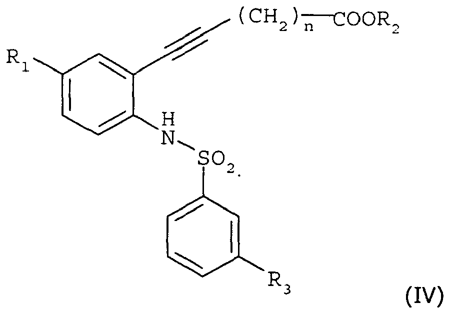 dans laquelle R₁, R₂, R₃, et n conservent la même signification que dans les composés de départ ;
c) cycliser le composé de formule (IV) ci-dessus, dans des conditions analogues à celles décrites pour réaliser l'étape (b) du procédé général précédent, pour obtenir le composé indolique de formule 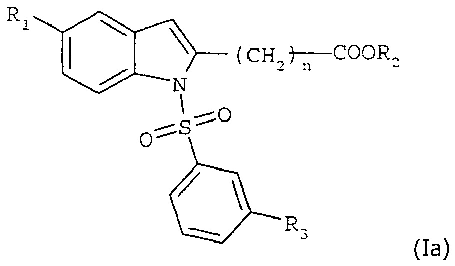 dans laquelle :
   R₁, R₂, R₃ et n conservent la même signification que dans le composé de départ ;
d) hydrolyser, si nécessaire, la fonction ester du composé de formule Ia, par exemple (dans le cas d'un ester t-butylique) par action d'un acide organique tel que l'acide trifluoroacétique dans un solvant tel que le dichlorométhane, selon des modes opératoires bien connus de l'homme du métier, pour obtenir le composé de formule I sous sa forme d'acide libre : 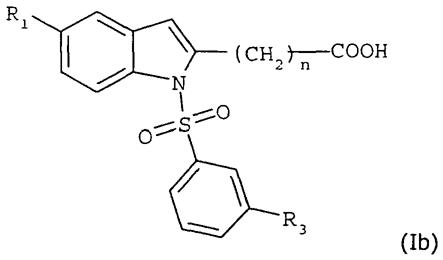 dans laquelle :
   R₁, R₂, R₃ et n conservent la même signification que dans le composé de départ.

Il est à noter que dans certaines conditions, les étapes b) et c) de ce procédé peuvent être avantageusement réalisées en une seule opération (procédé dit « one pot »).

Le composé de formule (II) dans laquelle R₁ représente un halogène ou un groupe trifluorométhyle, R₂ représente un groupe alkyle en C₁-C-₄ et n représente 3 ou 4, peut être obtenu par réaction d'une ortho-iodoaniline de formule 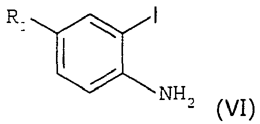 avec un ester d'acide alcynoïque de formule 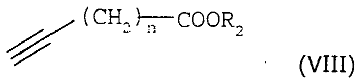 dans laquelle :
R₂ représente un groupe alkyle en C₁-C₄,
n = 3 ou 4 ;
en présence d'iodure cuivreux, d'un catalyseur à base de palladium tel que par exemple le chlorure de bis(triphénylphosphine) palladium, et d'une base organique comme par exemple la triéthylamine, dans un solvant comme par exemple le diméthylformamide (DMF) à une température comprise entre la température ambiante et 80 °C pendant 1 à 12 heures.

L'ester d'acide alcynoïque de formule 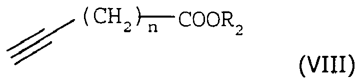 dans laquelle :
R₂ représente un groupe alkyle en C₁-C₄,
n = 3 ou 4
peut être obtenu au départ de l'acide alcynoïque correspondant par action successive du chlorure d'oxalyle, puis d'un alcoolate métallique de formule R₂OM, dans laquelle M représente un métal alcalin tel que par exemple le sodium ou le potassium.

Les composés de l'invention sous forme de sels d'un acide de formule (Ib) avec une base minérale ou organique, peuvent être obtenus de façon classique, en utilisant les méthodes bien connues de l'homme de métier, par exemple en mélangeant des quantités stoechiométriques de l'acide de formule (Ib) et de la base dans un solvant, tel que par exemple l'eau ou un mélange hydroalcoolique, et en lyophilisant ensuite la solution obtenue.

Dans certaines des étapes réactionnelles décrites ci-dessus, il est possible de remplacer avantageusement les méthodes de chauffage traditionnelles par un chauffage au moyen de micro-ondes en utilisant des réacteurs adaptés à ce mode de réaction. Dans ce cas, l'homme du métier comprendra que les durées de "chauffage" seront considérablement réduites, par comparaison aux durées nécessaires avec un chauffage classique.

Les exemples suivants de préparation de composés selon la formule (I) permettront de mieux comprendre l'invention.

Dans ces exemples, qui ne sont pas limitatifs de la portée de l'invention, on désigne par « préparation » les exemples décrivant la synthèse de composés intermédiaires et par « exemples » ceux décrivant la synthèse de composés de formule (I) selon l'invention.

Les abréviations suivantes ont été utilisées :
- mM : millimole,
- THF : tétrahydrofurane,
- DMF : diméthylformamide,
- DCM : dichlorométhane.

Les points de fusion sont mesurés au banc Kofler et les valeurs spectrales de Résonance Magnétique Nucléaire sont caractérisées par le déplacement chimique calculé par rapport au TMS (tétraméthylsilane), par le nombre de protons associés au signal et par la forme du signal (s pour singulet, d pour doublet, t pour triplet, q pour quadruplet, m pour multiplet). La fréquence de travail et le solvant utilisé sont indiqués pour chaque composé.

La température ambiante est de 20°C ± 5°C.

### PREPARATION 1

### Acide 6-[2-(((3-(1-méthyléthyl)phényl)sulfonyl)amino]-5-(trifluoro-méthyl)phényl]-5-hexynoïque, méthyl ester

On a préparé une solution de 42,90 g (150,39 mM) d'ester méthylique de l'acide 6-[2-amino-5-(trifluorométhyl)phényl]-5-hexynoïque dans 500 mL de pyridine et on a ajouté 37,90 g (173,29 mM) de chlorure de 3-(1-méthyléthyl)benzènesulfonyle. Le mélange a été agité pendant 15 heures à température ambiante puis versé sur un mélange de glace et d'acide chlorhydrique. Le mélange acide obtenu a été extrait trois fois par de l'acétate d'éthyle. Les phases organiques rassemblées ont été séchées sur sulfate de magnésium et concentrées sous pression réduite. L'huile résiduelle a été purifiée par chromatographie sur gel de silice en éluant à l'aide d'un mélange cyclohexane/acétate d'éthyle (9/1 ; v/v). On a ainsi obtenu 29,09 g du composé attendu sous forme d'une huile ocre (rendement = 41 %).
1H RMN (DMSOd6, 250 MHz) δ =1,12 (d, J=6,9, 6H), 1,76 (q, J=7,0, 2H), 2,40 (t, J=7,0, 2H), 2,44 (t, J=7,0, 2H), 2,92 (q, J=6,9, 1H), 3,62 (s, 3H), 7,47-7,51 (m, 4H), 7,62-7,66 (m, 3H), 9,68 (s, 1H).

### EXEMPLE 1

### Acide 1-[[3-(1-méthyléthyl)phényl]sulfonyl]-5-(trifluorométhyl)-1H indole-2-butanoïque, méthyl ester

On a préparé une solution de 28,12 g (60,15 mM) d'ester obtenu selon la préparation 1 dans 250 mL de 1,2-dichloroéthane et on a ajouté 12,49 g (62,55 mM) d'acétate de cuivre (cuivrique) monohydraté. On a placé le mélange sous azote et on l'a porté à reflux sous agitation pendant environ 15 heures. Le milieu réactionnel a été filtré et le résidu solide de filtration a été lavé sur le filtre au DCM. Les filtrats rassemblés ont été concentrés sous pression réduite. On a ainsi obtenu 27,70 g du composé attendu sous forme de cristaux beige (rendement = 99 %).
F = 115°C.

### EXEMPLE 2

### Acide 1-[[3-(1-méthyléthyl)phényl]sulfonyl]-5-(trifluorométhyl)-1H-indole-2-butanoïque

On a mélangé 27,50 g (58,82 mM) d'ester obtenu selon l'exemple 1 avec 450 mL de THF, et on a ajouté 4,23 g (176,47 mM) d'hydroxyde de lithium dans 100 mL d'eau. Le mélange a été agité pendant environ 15 heures à température ambiante puis refroidi à 0°C. On a alors ajouté progressivement 180 mL d'acide chlorhydrique N sous bonne agitation. La phase organique a été séparée et une moitié du solvant a été évaporée à froid sous pression réduite. Le résidu d'évaporation a été extrait trois fois par du dichlorométhane. Les phases organiques rassemblées ont été séchées sur sulfate de magnésium, filtrées et concentrées sous pression réduite. On a ainsi obtenu 26,22 g du produit attendu sous forme d'une poudre blanche (rendement = 98 %).
F = 160°C.

### EXEMPLE 2a

### Acide 1-[[3-(1-méthyléthyl)phényl]sulfonyl]-5-(trifluorométhyl)-1H-indole-2-butanoïque, sel de sodium

On a mélangé 68 mg (0,15 mM) d'acide obtenu selon l'exemple 2 en solution dans 4 mL de tétrahydrofurane avec 6 mg (0,15 mM) d'hydroxyde de sodium en solution dans 3 mL d'eau. Le mélange a été agité 6 heures à température ambiante puis concentré sous pression réduite. On a ainsi obtenu 65 mg du sel attendu sous forme de poudre cristalline blanche (rendement = 91 %).
F = 231 °C.

### EXEMPLE 2b

### Acide 1-[[3-(1-méthyléthyl)phényl]sulfonyl]-5-(trifluorométhyl)-1H-indole-2-butanoïque, sel de pipérazine

On a dissous 400 mg (0,88 mM) d'acide obtenu selon l'exemple 2 dans 10 mL de tétrahydrofurane et on a ajouté 76 mg (0,88 mM) de pipérazine. Le mélange réactionnel a été agité une nuit à température ambiante puis concentré sous pression réduite. On a ainsi obtenu 400 mg du sel attendu sous forme de poudre cristalline blanche (rendement = 46 %).
F = 147°C.

### EXEMPLE 2c

### Acide 1-[[3-(1-méthyléthyl)phényl]sulfonyl]-5-(trifluorométhyl)-1H-indole-2-butanoïque, sel de tris(hydroxyméthyl)aminométhane

On a dissous 400 mg (0,88 mM) d'acide obtenu selon l'exemple 2 dans 10 mL de tétrahydrofurane et on a ajouté 106,85 mg (0,88 mM) de tris(hydroxyméthyl)-aminométhane. On a ajouté 3 mL d'eau afin d'obtenir une solution. Le mélange réactionnel a été agité une nuit à température ambiante puis concentré sous pression réduite. Le résidu a été repris trois fois avec du méthanol en chassant ensuite le solvant sous pression réduite. On a ainsi obtenu 480 mg du sel attendu sous forme de poudre cristalline blanche (rendement = 95 %).
F = 126 °C.

### PREPARATION 2

### Acide 6-[5-chloro-2-[[[3-(1-méthyléthyl)phényl]sulfonyl]amino]phényl]-5-hexynoïque, méthyl ester

En opérant de façon analogue à la préparation 1, au départ d'ester méthylique de l'acide 6-(2-amino-5-chlorophényl)-5-hexynoïque, on a obtenu le composé attendu sous forme d'une huile marron (rendement = 96 %).
1H RMN (DMSOd₆, 300 MHz) δ =1,13 (d, J=6,9, 6H) 1,71 (q, J=7,1, 2H), 2,33 (t, J=7,1, 2H), 2,42 (t, J=7,4, 2H), 2,91 (q, J=6,9, 1H), 3,61 (s, 3H), 7,26 (d, J=7,3, 1H), 7,34-7,40 (m, 3H), 7,49-7,57 (m, 2H), 7,76-7,78 (m, 1H), 9,68 (s, 1H).

### EXEMPLE 3

### Acide 1-[[3-(1-méthyléthyl)phényl]sulfonyl]-5-chloro-1H-indole-2-butanoïque, méthyl ester

On a préparé une solution de 0,3 g (0,69 mM) d'ester obtenu selon la préparation 2 dans 13 mL de 1,2-dichloréthane et on a ajouté 0,21 g (1,05 mM) d'acétate cuivrique monohydraté. Le mélange réactionnel a été irradié au four micro-ondes à 120°C pendant 15 minutes, puis refroidi et filtré. Le résidu sur le filtre a été lavé au DCM puis le filtrat a été concentré sous pression réduite. Le produit brut a été purifié par chromatographie sur gel de silice en éluant à l'aide d'un mélange cyclohexane/acétate d'éthyle (9/1 ; v/v). On a ainsi obtenu 0,23 g du composé attendu sous forme d'un solide beige (rendement = 77 %).
F = 94 - 97°C.
1H RMN (DMSOd₆, 250MHz) δ = 1,11 (d, J=6,9, 6H), 1,95 (q, J=7,4, 2H), 2,42 (t, J=7,4, 2H), 2,94 (q, J=7,4, 1H), 3,02 (t, J=7,4, 2H), 3,59 (s, 3H), 6,61 (s, 1H), 7,32 (dd, J=2,2 et 8,9, 1H), 7,47 (t, J=7,9, 1H), 7,56-7,63 (m, 4H), 8,06 (d, J=8,9, 1H).

### EXEMPLE 4

### Acide 1-[[3-(1-méthyléthyl)phényl]sulfonyl]-5-chloro-1H-indole-2-butanoïque

En opérant de façon analogue à l'exemple 2, au départ du composé obtenu selon l'exemple 3, on a obtenu le produit attendu sous forme d'un solide beige foncé (rendement = 93 %).
F = 128°C.

### REPARATION 3

### Acide 6-heptynoïque, 1,1-diméthyléthyl ester

On a dissous 8,00 g (63,41 mM) d'acide 6-heptynoïque dans un mélange de 137 mL de dichlorométhane anhydre et de 0,70 mL de diméthylformamide anhydre. On a ajouté goutte à goutte 16,10 g (126,83 mM) de chlorure d'oxalyle. Le milieu réactionnel a été agité pendant 1 heure à température ambiante sous atmosphère d'azote, puis évaporé sous atmosphère d'azote. Le produit résiduel a été repris par 137 mL de tétrahydrofurane. Le mélange a été refroidi à 0°C et on a additionné par fraction 14,23 g (126,83 mM) de tert-butoxyde de potassium. Le milieu réactionnel a été maintenu sous agitation à température ambiante pendant une heure. On a ensuite ajouté 200 g de glace et 200 mL d'eau. Le mélange a été extrait par 3 fois 200 mL d'éther puis les phases organiques rassemblées ont été séchées sur sulfate de magnésium et concentrées sous pression réduite. On a ainsi obtenu 7,46 g du composé attendu sous forme d'une huile marron (rendement = 65 %).
1H RMN (DMSOd6, 250MHz) δ = 1,40 (s, 9H), 1,40-1,45 (m, 4H), 2,13-2,22 (m, 4H), 2,75 (t, J=2,7, 1H).

### PREPARATION 4

### Acide 7-[2-amino-5-(trifluorométhyl)phényl]-6-heptynoïque, 1,1-diméthyléthyl ester

On a préparé une solution de 9,78 g (34,07 mM) de 2-iodo-4-(trifluorométhyl)aniline et de 7,45 g (40,89 mM) de l'ester de l'acide 6-heptynoïque obtenu selon la préparation 3 dans 136 mL de triéthylamine. On a ajouté 1,20 g (1,70 mM) de dichloro-bis(triphénylphosphine)palladium et 0,3 g mg (1,70 mM) d'iodure cuivreux. Le mélange réactionnel a été agité et chauffé à reflux sous atmosphère d'azote pendant 3 heures, puis il a été concentré sous pression réduite. Le résidu d'évaporation a été repris dans l'acétate d'éthyle et lavé avec une solution d'hydrogénocarbonate de sodium (env 1 M dans l'eau), puis avec de l'acide chlorhydrique 1 N et enfin avec de l'eau distillée. La phase organique a été séchée sur sulfate de magnésium, filtrée et concentrée sous pression réduite. On a ainsi obtenu 12,38 g du composé attendu sous forme d'une huile marron (rendement = 71 %).
¹H RMN (DMSOd₆, 250MHz) δ = 1,40 (s, 9H), 1,53-1,68 (m, 4H), 2,24 (t, J=8,4, 2H), 2,48 (t, J=8,1_{,} 2H), 5,93 (s, 2H), 6,78 (d, J=10,2, 1H), 7,28-7,33 (m, 2H).

### PREPARATION 5

### Acide 5-trifluorométhyl-1H-indole-2-pentanoïque, 1,1-diméthyléthyl ester

On a préparé une solution de 7,63 g (22,35 mM) d'ester t-butylique de l'acide 7-[2-amino-5-(trifluorométhyl)phényl]-6-heptynoïque dans 44,70 mL de 1,2-dichloroéthane et on a ajouté 6,69 g (33,52 mM) d'acétate cuivrique monohydraté. On a porté le mélange à reflux sous agitation pendant 48 heures. Le milieu réactionnel a été filtré sur filtre nylon puis le filtrat a été concentré sous pression réduite. Le produit brut a été purifié par chromatographie sur gel de silice en éluant à l'aide d'un mélange cyclohexane/acétate d'éthyle (9/1 ; v/v). On a ainsi obtenu 3,42 g du composé attendu sous forme d'une poudre jaune (rendement = 45 %).
1H RMN (DMSOd₆, 250MHz) δ = 1,38 (s, 9H), 1,51-1,57 (m, 2H), 1,67-1,73 (m, 2H), 2,23 (t, J=8,4, 2H), 2,75 (t, J=8,7, 2H), 6,31 (s, 1H), 7,28 (dd, J=2,1 et 10,2, 1H), 7,44 (d, J=10,2, 1H), 7,79 (s, 1H).

### EXEMPLE 5

### Acide 1-[[3-(1-méthyléthyl)phényl]sulfonyl]-5-(trifluorométhyl)-1H-indole-2-pentanoïque, 1,1-diméthyléthyl ester

On a ajouté 46,87 mg (1,17 mM) d'hydrure de sodium (à 60 % dans l'huile) à une solution de 200,00 mg (0,59 mM) d'ester obtenu selon la préparation 5 dans 0,5 mL de DMF, à 0 °C. Ce mélange a été agité pendant 5 minutes et on a ajouté, toujours à 0°C, une solution de 192,20 mg (0,88 mM) de chlorure de 3-(1-méthyléthyl)benzènesulfonyle dans 0,5 mL de DMF. Le mélange a été agité pendant 3 heures à température ambiante, puis on a ajouté une solution de chlorure d'ammonium pour neutraliser les traces d'hydrure de sodium. Le mélange a été extrait avec du dichlorométhane. La phase organique a été concentrée sous pression réduite, puis le milieu réactionnel ainsi obtenu a été mis en réaction dans l'étape suivante sans purification.

### EXEMPLE 6

### Acide 1-[[3-(1-méthyléthyl)phényl]sulfonyl]-5-(trifluorométhyl)-1H-indole-2-pentanoïque

On a préparé une solution de 200,00 mg (0,38 mM) d'ester obtenu selon l'exemple 5 dans 1 mL de DCM et on a ajouté 1 mL d'acide trifluoroacétique. Le milieu réactionnel a été agité à température ambiante pendant 3 heures puis repris par du DCM et concentré sous pression réduite. Le produit brut a été purifié par chromatographie sur gel de silice en éluant à l'aide d'un mélange cyclohexane/acétate d'éthyle (6/4 ; v/v). On a ainsi obtenu 50,00 mg du composé attendu sous forme d'une poudre blanc-cassé (rendement = 26 %).
F = 119°C.

### PREPARATION 6

### 3-(1,1-diméthyléthyl)-N-[2-iodo-4-(trifluorométhyl)phényl]-benzènesulfonamide

On a préparé une solution de 1,03 g (3,59 mM) de 2-iodo-4-(trifluorométhyl)aniline dans 5 mL de pyridine et on a ajouté 1,00 g (4,31 mM) de chlorure de 3-(1,1-diméthyléthyl)-benzènesulfonyle. Le mélange réactionnel a été ensuite agité à température ambiante pendant 4 heures. Le milieu réactionnel a été lavé à l'acide chlorhydrique 1N et extrait deux fois à l'acétate d'éthyle. La phase organique a été séchée sur sulfate de magnésium puis filtrée et concentrée sous pression réduite. Le produit brut a été purifié par chromatographie sur gel de silice en éluant à l'aide d'un mélange cyclohexane/acétate d'éthyle (gradient de 100/0 à 90/10 ; v/v). On a ainsi obtenu 730 mg du composé attendu sous forme d'une poudre cristalline blanche (rendement = 42 %).
F = 111°C.

### EXEMPLE 7

### Acide 1-[[3-(1,1-diméthyléthyl)phényl]sulfonyl]-5-(trifluorométhyl)-1H-indole-2-butanoïque, méthyl ester

On a préparé sous azote un mélange de 250 mg (0,52 mM) du composé obtenu selon la préparation 6, 4,93 mg (0,03 mM) d'iodure cuivreux, 9,08 mg (0,01 mM) de bis(triphénylphosphine)dichloropalladium et 3 mL de triéthylamine. Le milieu réactionnel a été agité à température ambiante pendant 10 minutes. On a ajouté 120,31 mg (0.95 mM) d'ester méthylique de l'acide 5-hexynoïque en solution dans 3 mL de diméthylformamide. Le mélange réactionnel a été chauffé à reflux pendant 3 heures puis lavé à l'eau et extrait par de l'acétate d'éthyle. La phase organique a été séchée sur sulfate de magnésium et concentrée sous pression réduite. Le produit brut a été purifié par chromatographie sur gel de silice en éluant à l'aide d'un mélange cyclohexane/acétate d'éthyle (95/5 ; v/v). On a ainsi obtenu ainsi 115 mg du produit attendu sous forme d'une poudre cristalline beige (rendement = 46 %).
F = 84°C.

### EXEMPLE 8

### Acide 1-[[3-(1,1-diméthyléthyl)phényl]sulfonyl]-5-(trifluorométhyl)-1H-indole-2-butanoïque

En opérant de façon analogue à l'exemple 2, au départ du composé obtenu selon l'exemple 7, on a obtenu le produit attendu sous forme d'une poudre blanche (rendement = 27 %).
F = 135 - 141°C.

### EXEMPLE 9

### Acide 1-[[3-(1,1-diméthyléthyl)phényl]sulfonyl]-5-(trifluorométhyl)-1H-indole-2-pentanoïque, méthyl ester

On a préparé sous azote un mélange de 57,93 g (119,87 mM) du composé obtenu selon la préparation 6 et de 350 mL de diméthylformamide et on a agité jusqu'à dissolution totale du produit. On a alors ajouté successivement 21,84 g (155,83 mM) d'ester méthylique de l'acide 4-pentynoïque, 1,14 g (5,99 mM) d'iodure cuivreux et 1,68 g (2,40 mM) de bis(triphénylphosphine)dichloropalladium. Ce mélange a été agité pendant 15 mn à température ambiante, puis on a ajouté goutte à goutte 174 mL de triéthylamine. Le milieu réactionnel a été chauffé pendant 14 heures à 80°C, refroidi, puis hydrolysé par 1 I d'eau et extrait par de l'acétate d'éthyle. La phase organique a été séchée sur sulfate de magnésium, filtrée et concentrée sous pression réduite. Le produit huileux obtenu a été dissous à 40°C dans l'éther isopropylique. La solution obtenue a été filtrée et concentrée sous pression réduite. Le produit obtenu a été recristallisé dans un mélange de 140 mL d'isopropanol et 60 mL d'eau. On a ainsi obtenu ainsi 46,51 g du produit attendu sous forme d'un solide blanc cassé (rendement = 78 %).
F = 77°C.

### EXEMPLE 10

### Acide 1-[[3-(1,1-diméthyléthyl)phényl]sulfonyl]-5-(trifluorométhyl)-1H-indole-2-pentanoïque

En opérant de façon analogue à l'exemple 2, au départ du composé obtenu selon l'exemple 9, on a obtenu le produit attendu sous forme d'un solide blanc cassé (rendement = 94 %).
F = 135 °C.

Les composés selon l'invention décrits ci-dessus ont été reportés dans le tableau suivant:

**TABLEAU I :**

| | | | | |
|---|---|---|---|---|
| 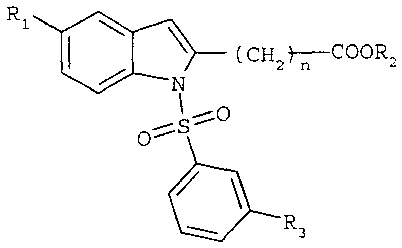 | | | | |

| **Ex** | **R₁** | **n** | **R₂** | **R₃** |
|---|---|---|---|---|
| 1 | 5-CF₃ | 3 | CH₃ | CF(CH₃)₂ |
| 2 | 5-CF₃ | 3 | H | CH(CH₃)₂ |
| 3 | 5-Cl | 3 | CH₃ | CH(CH₃)₂ |
| 4 | 5-Cl | 3 | H | CH(CH₃)₂ |
| 5 | 5-CF₃ | 4 | C(CH₃)₃ | CH(CH₃)₂ |
| 6 | 5-CF₃ | 4 | H | CH(CH₃)₂ |
| 7 | 5-CF₃ | 3 | CH₃ | C(CH₃)₃ |
| 8 | 5-CF₃ | 3 | H | C(CH₃)₃ |
| 9 | 5-CF₃ | 4 | CH₃ | C(CH₃)₃ |
| 10 | 5-CF₃ | 4 | H | C(CH₃)₃ |

### Activité pharmacologique

Les composés de l'invention ont été soumis à des tests biologiques de façon à évaluer leur potentiel à traiter ou prévenir certaines pathologies neurodégénératives.

Dans un premier temps, on a mesuré, par un test *in vitro,* l'aptitude des composés selon l'invention à se comporter en activateur des hétérodimères formés par le récepteur nucléaire NURR-1 et les récepteurs nucléaires RXR.

Un test de transactivation a été utilisé comme test de screening primaire. Des cellules Cos-7 ont été co-transfectées avec un plasmide exprimant une chimère du récepteur humain NURR-1-Gal4, un plasmide exprimant le récepteur humain RXR (récepteur RXRα ou RXRγ) et un plasmide rapporteur 5Ga14pGL3-TK-Luc. Les transfections ont été réalisées à l'aide d'un agent chimique (Jet PEI).

Les cellules transfectées ont été distribuées dans des plaques 384 puits et laissées au repos pendant 24 heures.

Au temps 24 heures le milieu de culture a été changé. Les produits à tester ont été ajoutés (concentration finale comprise entre 10⁻⁴ et 3.10⁻¹⁰ M) dans le milieu de culture. Après une nuit d'incubation, l'expression de luciférase a été mesurée après addition de « SteadyGlo » selon les instructions du fabricant (Promega).

L'acide 4-[[6-méthyl-2-phényl-5-(2-propényl)-4-pyrimidinyl]amino]-benzoïque (nommé XCT0135908) à 2.10⁻⁵ M (agoniste RXR) a été utilisé comme référence.

Les niveaux d'induction ont été calculés par rapport à l'activité basale de chaque hétérodimère. Les résultats ont été exprimés en pourcentage du niveau d'induction par rapport au niveau d'induction obtenu avec la référence (le niveau d'induction de la référence est arbitrairement égal à 100 %).

Les composés selon l'invention présentent un taux d'induction allant jusqu'à 104 % (NURR1/RXRα) et 88 % (NURR1/RXRγ) et des EC50 allant jusqu'à 26 nM (NURR1/RXRα) et 20 nM (NURR1/RXRγ).

Certains composés selon l'invention présentent une EC₅₀ inférieure à 100 nM, notamment sur l'hétérodimère NURR-1/RXRα.

A titre d'exemple, parmi les composés selon l'invention, on obtient les résultats comparatifs exprimés en pourcentage par rapport à un composé de référence activateur NURR-1/RXR (XCT0135908) suivants :

| Composé | hNurr1_RXRγFL | | hNurr1_RXRαFL | |
|---|---|---|---|---|
| | EC₅₀(nM) | Eff(%) | EC₅₀(nM) | Eff(%) |
| Exemple 2 | 113 | 79 | 73 | 86 |
| Exemple 8 | 20 | 70 | 26 | 100 |
| Exemple 10 | 77 | 88 | 55 | 104 |
| Exemple comparatif * | 1108 | 74 | 571 | 75 |

| | | | | |
|---|---|---|---|---|
| * : exemple 76 de la demande de brevet WO 2007/026097 Eff signifie : efficacité en % par rapport à la référence XCT0135908 | | | | |

A titre de comparaison, on a aussi étudié l'exemple 76 de la demande de brevet WO 2007/026097, de structure relativement proche des composés selon l'invention, pour lequel les résultats montrent que la concentration à laquelle le composé donne la moitié de l'efficacité maximale (EC₅₀) est au moins 10 fois supérieure à celle des composés décrits dans l'invention.

Une première série de tests *in vivo* a été pratiquée avec quelques composés selon l'invention, dans le but de déterminer leur profil pharmacocinétique plasmatique et cérébral chez la souris C57BI6 male et vérifier ainsi que les composés passent la barrière hématoencéphalique.

Le protocole suivant a été utilisé.

Des souris males C57BI6 (25-30 g) provenant des établissements Janvier, Le Genest-St-Isle, France ont été utilisées pour cette étude (12 souris par dose).

Les animaux ont été nourris avec de la nourriture standard pour rongeurs (Purina Mills, St. Louis, MO), placés dans des cages et soumis à des cycles lumière/obscurité de 12h/12h, la température de pièce étant maintenue à 22± 2°C et le taux d'humidité à 55±10 %.

Les souris n'ont pas été mises à jeun avant l'administration. L'eau a été fournie à volonté durant toute l'étude.

Le composé à tester a été administré par voie orale à 10 mg/kg.

Pour l'administration orale à 10 mg/kg, les animaux ont été gavés avec 10 mL/kg d'une suspension du composé à tester, préparée dans de la méthylcellulose 400 cp 1 %.

Les animaux ont été sacrifiés sous anesthésie aux temps 15 mn, 30 mn, 1 h, 3 h, 6 h et 8 h après gavage.

A chaque temps, et sur chaque animal sacrifié, le sang a été collecté et le cerveau a été prélevé.

1 mL de sang collecté dans des tubes de 1,5 mL contenant 20 µl d'anticoagulant évaporé (solution d'héparinate de sodium à 1000 UI/mL) a été centrifugé à 4500 g pendant 3 min pour obtenir environ 400 µL de plasma. Le plasma a été réparti en 2 aliquotes de 200 µL qui ont été conservés à -20°C jusqu'à extraction par précipitation protéique puis analyse par chromatographie liquide couplée à la spectrométrie de masse tandem (LC-MS/MS) pour la quantification du composé testé.

Les cerveaux ont été plongés dans l'azote liquide directement après le prélèvement, puis conservés à -20°C pour analyse. Les cerveaux ont ensuite été broyés en présence de mélange aqueux/solvant organique afin d'obtenir un homogénat. Ces homogénats ont ensuite été centrifugés et le composé testé a été extrait à partir du surnageant obtenu, par une extraction liquide-liquide, puis quantifié par LC-MS/MS.

Les paramètres pharmacocinétiques ont été déterminés à partir d'une approche non-compartimentale sous Excel. L'aire sous la courbe (AUC₀₋ₜ) a été déterminée par la méthode trapézoïdale linéaire.

A titre d'exemple, avec les composés des exemples 2, 8 et 10, on a obtenu les résultats suivants :

| Composé | Données PK après administration orale : 10 mg/kg chez les souris | |
|---|---|---|
| | AUC _{cerveau} | Ratio AUC_{cerveau}/AUCₚₗₐₛₘₐ |
| Exemple 2 | 3318 | 0,67 |
| Exemple 8 | 2371 | 0,87 |
| Exemple 10 | 1689 | 0,80 |

Une seconde série de tests *in vivo* a été pratiquée avec les composés selon l'invention, dans le but de vérifier que les molécules possèdent bien l'effet neuroprotecteur attendu.

Le composé de l'exemple 2, a été testé sur un modèle de souris traitées par la 1-méthyl-4-phényl-1,2,3,6-tétrahydropyridine (MPTP) afin de confirmer son activité potentielle. La MPTP est une neurotoxine qui provoque les symptômes permanents de la maladie de Parkinson en détruisant certains neurones dans la substantia nigra du cerveau. Le protocole suivant a été utilisé.

Des souris mâles C57BL6/J âgées de 10-12 semaines au début des études, ont été réparties par groupe de 8 animaux. Le composé a été administré par voie orale, 2 fois par jour pendant 11 jours au total. L'administration a commencé 3 jours avant le traitement avec la toxine MPTP à 20 ou 25 mg/kg. La MPTP a été administrée une fois par jour par injection intra-péritonéale pendant 5 jours. L'administration du composé à tester a été poursuivie pendant 3 jours après le traitement à la MPTP. Un groupe de souris a reçu le véhicule seul (solution de méthylcellulose à 0,5%). Les animaux ont été euthanasiés après le dernier gavage et le striatum a été prélevé. La dopamine a été extraite du striatum et la quantité de dopamine (DA) exprimée en ng par g de striatum (moyenne ± SEM) a été mesurée par chromatographie liquide haute performance (CLHP) avec détection électrochimique.

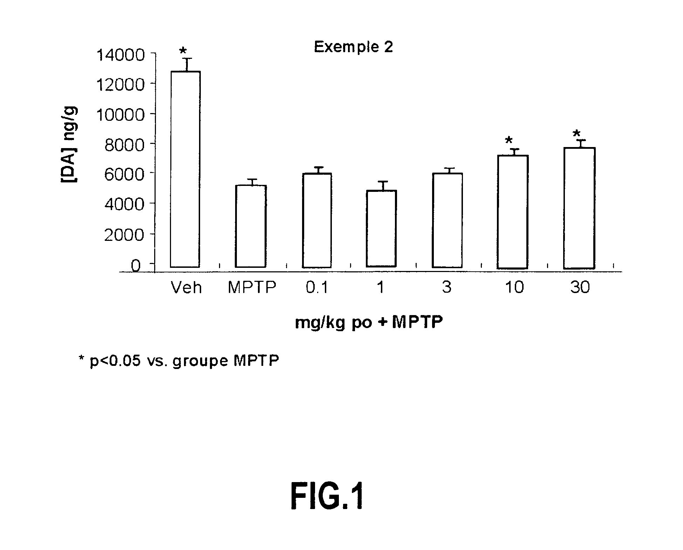

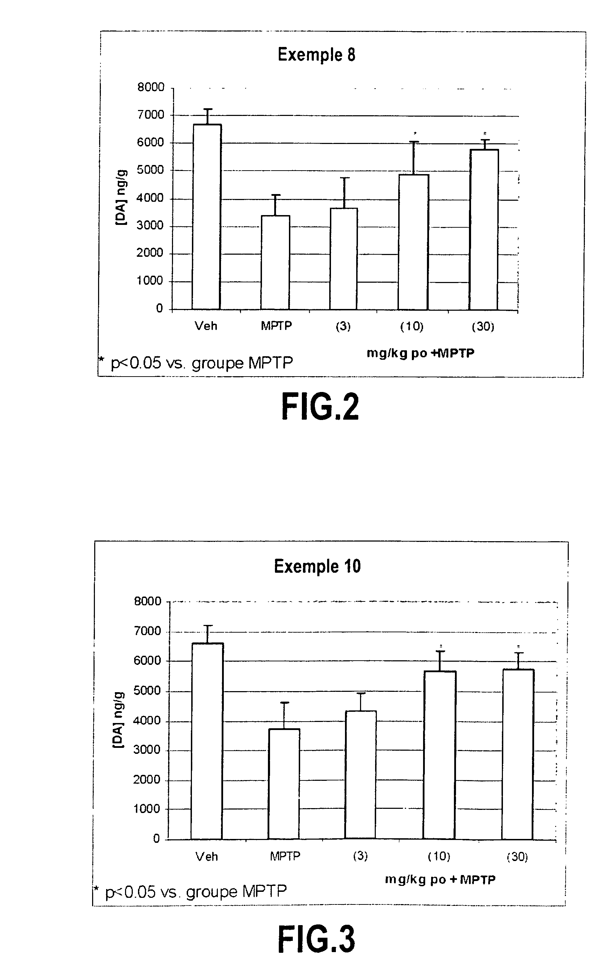

Les résultats obtenus ont été reportés aux figures 1 à 3 annexées.

Ces résultats montrent que l'administration de la MPTP provoque une diminution caractéristique du niveau de dopamine dans le striatum et que les composés selon les exemples 2, 8 et 10 diminuent de manière dose dépendante l'action de la MPTP, une toxine qui provoque un syndrome parkinsonien.

On observe ainsi un effet significatif aux doses de 10 et 30 mg /kg : les composés de l'invention, administrés par voie orale, sont capable de rétablir l'activité dopaminergique inhibée par la MPTP au niveau du cerveau.

De tels composés, qui traversent la barrière hématoencéphalique et possèdent un effet favorable à la communication entre les neurones, peuvent avantageusement être utilisés en tant que principe actif d'un médicament destiné au traitement de la maladie de Parkinson.

Ces résultats *in vitro* et *in vivo* montrent que les composés de l'invention sont capables de modifier les mécanismes de la maladie sur certains modèles cellulaires et animaux et de stopper le processus dégénératif en générant des agents neuroprotecteurs permettant de lutter contre la mort cellulaire des neurones dopaminergiques. Ils confirment donc l'intérêt de ces composés pour leur utilisation en tant que principes actifs de médicaments destinés à la prévention ou au traitement des maladies neurodégénératives, et plus particulièrement, de la maladie de Parkinson.

L'invention concerne également une composition pharmaceutique contenant, en tant que principe actif, au moins un composé de la formule (I), ou l'un de ses sels pharmaceutiquement acceptable.

Selon un autre aspect, la présente demande vise à couvrir l'utilisation d'une telle composition pharmaceutique pour la prévention ou le traitement des maladies dans lesquelles le récepteur NURR-1 est impliqué, notamment les maladies neurodégénératives, et plus particulièrement la maladie de Parkinson.

Ces compositions pharmaceutiques peuvent être préparées de façon classique, à l'aide d'excipients pharmaceutiquement acceptables afin d'obtenir des formes administrables par voie parentérale ou, de préférence, par voie orale, par exemple des comprimés ou des gélules.

Dans le cas de formes injectables, on utilisera avantageusement les composés de formule (I) sous forme de sels solubles dans un milieu aqueux. Comme indiqué précédemment, les sels sont préférentiellement formés entre un composé de formule (Ib) (acide) et une base non toxique pharmacologiquement acceptable. La formulation peut être soit une solution du composé dans un milieu aqueux isotonique en présence d'excipients solubles, soit un lyophilisat du composé auquel le solvant de dilution est ajouté de façon extemporanée. Ces préparations pourront être injectées sous forme de perfusion ou en bolus en fonction des besoins du patient.

De façon pratique, en cas d'administration du composé par voie parentérale, la posologie quotidienne chez l'homme sera de préférence comprise entre 2 et 250 mg.

Les préparations administrables par voie orale seront de préférence présentées sous forme d'une gélule ou d'un comprimé contenant le composé de l'invention broyé finement ou mieux, micronisé, et mélangé avec des excipients connus de l'homme du métier, tels que par exemple du lactose, de l'amidon prégélatinisé et du stéarate de magnésium.

A titre d'exemple, on a granulé un mélange constitué de 500 g du composé de l'exemple 2 finement broyé, 500 g d'amidon prégélatinisé, 1250 g de lactose, 15 g de laurylsulfate de sodium et 235 g de polyvinylpyrrolidone. Ce mélange granulé a ensuite été additionné à 20 g de stéarate de magnésium et 80 g de cellulose microcristalline et le mélange obtenu a été réparti après broyage et tamisage dans des gélules de 260 mg. On a ainsi obtenu des gélules contenant chacune 50 mg de principe actif.

De façon pratique, en cas d'administration du composé par voie orale, la posologie quotidienne chez l'homme sera de préférence comprise entre 5 et 500 mg.

## Revendications

1. Composé dérivé de l'indole, notamment utile en thérapeutique, **caractérisé en ce qu'**il est choisi parmi
i) les composés de formule (I) 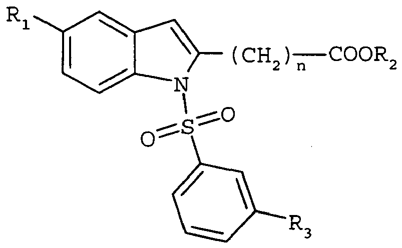 dans laquelle :
R₁ représente un halogène ou un groupe trifluorométhyle,
R₂ représente un atome d'hydrogène ou un groupe alkyle en C₁-C₄,
R₃ représente un groupe isopropyle (1-méthyléthyl) ou un groupe *tertio-butyle* (1,1-diméthyléthyl),
n = 3 ou 4
ii) les sels pharmaceutiquement acceptables desdits composés de formule (I).

2. Composé selon la revendication 1, **caractérisé en ce que** dans la formule (I) précitée :
R₃ représente un groupe isopropyle.

3. Composé selon la revendication 1, **caractérisé en ce que** dans la formule (I) précitée :
R₃ représente un groupe t-butyle.

4. Composé selon l'une des revendications 1 à 3, **caractérisé en ce que** dans la formule (I) précitée :
R₂ représente un atome d'hydrogène.

5. Composition pharmaceutique, **caractérisée en ce qu'**elle comprend au moins un composé selon l'une des revendications 1 à 4, en tant que substance active et au moins un excipient pharmaceutiquement acceptable.

6. Composé dérivé d'indole selon l'une quelconque des revendications 1 à 4 pour son utilisation en tant que substance thérapeutiquement active.

7. Composé dérivé d indole selon l'une quelconque des revendications 1 à 4 pour son utilisation dans le traitement ou la prévention des maladies neurodégénératives.

8. Composé selon la revendication 7, **caractérisée en ce que** la maladie précitée est la maladie de Parkinson.

## Claims

1. Compound derived from indole, in particular useful in therapy, **characterized in that** it is selected from
i) compounds of formula (I) 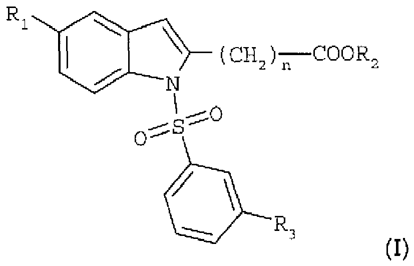 in which
R₁ represents a halogen or a trifluoromethyl group,
R₂ represents a hydrogen atom or a C₁-C₄ alkyl group,
R₃ represents an isopropyl (1-methylethyl) group or a tert-butyl (1,1-dimethylethyl) group,
n = 3 or 4
and
ii) pharmaceutically acceptable salts of said compounds of formula (I).

2. Compound according to Claim 1, **characterized in that** in formula (I) above R₃ represents an isopropyl group.

3. Compound according to Claim 1, **characterized in that** in formula (I) above R₃ represents a *tert-*butyl group.

4. Compound according to any of Claims 1 to 3, **characterized in that** in formula (I) above R₂ represents a hydrogen atom.

5. Pharmaceutical composition, **characterized in that** it comprises at least one compound according to any of Claims 1 to 4 as an active substance, and at least one pharmaceutically acceptable excipient.

6. Compound derived from indole according to any of Claims 1 to 4 for its use as a therapeutically active substance.

7. Compound derived from indole according to any of Claims 1 to 4 for its use in the treatment or prevention of neurodegenerative diseases.

8. Compound according to Claim 7, **characterized in that** the aforementioned disease is Parkinson's disease.

## Patentansprüche

1. Von Indol abgeleitete Verbindung, die insbesondere in der Therapie nützlich ist, **dadurch gekennzeichnet, daß** sie ausgewählt ist aus
i) den Verbindungen der Formel (I) 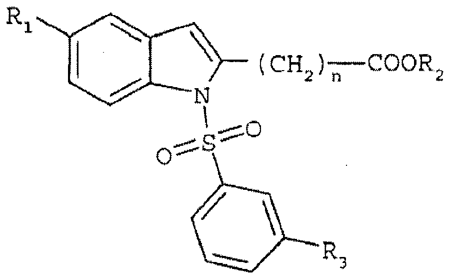 worin:
R₁ ein Halogen oder eine Trifluormethylgruppe darstellt,
R₂ ein Wasserstoffatom oder eine C₁-C₄-Alkylgruppe darstellt,
R₃ eine Isopropylgruppe (1-Methylethyl) oder eine tert-Butylgruppe (1,1-Dimethylethyl) darstellt,
n = 3 oder 4
ii) den pharmazeutisch verträglichen Salzen der Verbindungen der Formel (I).

2. Verbindung nach Anspruch 1, **dadurch gekennzeichnet, daß** in der vorstehend genannten Formel (I):
R₃ eine Isopropylgruppe darstellt.

3. Verbindung nach Anspruch 1, **dadurch gekennzeichnet, daß** in der vorstehend genannten Formel (I):
R₃ eine t-Butylgruppe darstellt.

4. Verbindung nach einem der Ansprüche 1 bis 3, **dadurch gekennzeichnet, daß** in der vorstehend genannten Formel (I):
R₂ ein Wasserstoffatom darstellt.

5. Pharmazeutische Zusammensetzung, **dadurch gekennzeichnet, daß** sie wenigstens eine Verbindung nach einem der Ansprüche 1 bis 4 als Wirkstoff sowie wenigstens einen pharmazeutisch verträglichen Hilfsstoff umfaßt.

6. Von Indol abgeleitete Verbindung nach einem der Ansprüche 1 bis 4, für ihre Verwendung als therapeutisch wirksame Substanz.

7. Von Indol abgeleitete Verbindung nach einem der Ansprüche 1 bis 4, für ihre Verwendung bei der Behandlung oder der Vorbeugung von neurodegenerativen Erkrankungen.

8. Verbindung nach Anspruch 7, **dadurch gekennzeichnet, daß** die vorstehend genannte Erkrankung die Parkinson-Krankheit ist.
